Europäisches Patentamt

European Patent Office

Office européen des brevets

(11) Publication number: **0 253 581 B1**

# EUROPEAN PATENT SPECIFICATION

(45) Date of publication of patent specification: **16.10.91**    (51) Int. Cl.5: **G01N 33/52**

(21) Application number: **87306094.1**

(22) Date of filing: **10.07.87**

(54) Analytical element having water-soluble polymers and determinations using same.

(30) Priority: **10.07.86 US 884237**

(43) Date of publication of application:
**20.01.88 Bulletin 88/03**

(45) Publication of the grant of the patent:
**16.10.91 Bulletin 91/42**

(84) Designated Contracting States:
**CH DE FR GB LI**

(56) References cited:
**EP-A- 0 209 378**
**JP-A-57 082 767**
**JP-A-61 004 963**
**US-A- 4 459 358**

(73) Proprietor: **EASTMAN KODAK COMPANY (a New Jersey corporation)**
**343 State Street**
**Rochester New York 14650(US)**

(72) Inventor: **Columbus, Richard Lewis, c/o EAST-MAN KODAK Co.**
**Pat. Dept., 343 State Street**
**Rochester, NY 14650(US)**
Inventor: **Palmer, Harvey John, c/o EASTMAN KODAK Co.**
**Pat. Dept., 343 State Street**
**Rochester, NY 14650(US)**
Inventor: **Sundberg, Michael William, c/o EASTMAN KODAK Co.**
**Pat. Dept., 343 State Street**
**Rochester, NY 14650(US)**

(74) Representative: **Nunney, Ronald Frederick Adolphe et al**
**Kodak Limited Patent Department Headstone Drive**
**Harrow Middlesex HA1 4TY(GB)**

## Description

This invention relates to clinical chemistry and to elements useful for the determination of analytes in aqueous liquids, such as biological fluids.

Competitive binding immunoassays, which take advantage of natural immunological reactions, have found widespread use as analytical techniques in clinical chemistry. Because of the specificity of the reactions, they are particularly advantageous in quantifying analytes which are present in very low concentration and cannot be adequately quantitated by chemical techniques. Such analytes (called ligands herein) include, for example, therapeutic drugs, narcotics, enzymes, hormones, proteins, etc. Several techniques have been devised for determining very low concentrations of ligands. For instance, a ligand may be labeled by various means to make it readily measurable. In competitive binding assay, a labeled ligand analog (identified as ligand analog herein) is placed in competition with unlabeled ligand for reaction with a fixed amount of the appropriate binding material (called a receptor herein). Unknown concentrations of the ligand can be determined from the measured signal of either the bound or unbound (i.e. free) ligand analog. The reaction proceeds as follows:

ligand + ligand analog + receptor $\rightleftarrows$ ligand-receptor + ligand analog-receptor.

Conventional labels include radioactive isotopes, enzymes, chromophores, fluorophores, stable free radicals, and enzyme cofactors, inhibitors and allosteric effectors.

Sensitivity is of prime importance due to the extremely low level of ligands to be measured. The first highly sensitive assays used radioactive isotopes as labels. Fluorescent or enzyme labels are currently preferred in most commercial immunoassays.

Competitive binding immunoassays can also be classified as either heterogeneous or homogeneous. Heterogeneous immunoassays require a separation of bound ligand analog from free ligand analog. This separation is necessary because the properties of bound and free analog are not significantly different. Homogeneous immunoassays do not require a separation step because the properties of the bound and free analogs are different enough so that they can be differentiated.

It is often necessary to keep components that react during the analysis (such as the ligand analog and the receptor in a competitive immunoassay or an enzyme label and its substrate) separate until the time at which the sample to be analyzed is contacted with the element. This insures that competition between the ligand and the ligand analog can be properly established so that equilibrium in the competitive reaction can be reached in a reasonable time and that formation of the detectable end product occurs in a manner relating to the amount of ligand in the sample.

A common technique for keeping components separate is to add one or more of the necessary components, such as the ligand analog, to the test sample just prior to contacting the sample with the analytical element. This step, however, is difficult, time-consuming, and introduces an additional opportunity for error. Thus, it is desirable to incorporate as many of the necessary components as possible in the analytical element itself.

US-A-4 459 358 discloses a multilayer element for the analysis of a liquid containing an analyte comprising a reaction layer which retains a predetermined amount of a diffusible reagent containing a detectable species, said reagent adapted to be rendered substantially non-diffusible by said analyte as a function of the concentration of the analyte. The reaction layer comprises a hydrophilic colloid which is permeable to a liquid sample containing the analyte, and which is swellable but insoluble to maintain wet strength integrity.

EP-A-0 209 378 (comprised in the state of the art by virtue of Article 54(3) EPC) describes a heterogeneous immunoassay utilizing horizontal separation in an analytical element.

In European Patent 66,648, there is described an immunoassay analytical element having a reaction layer containing proteins (receptors) capable of causing competitive immune reaction. If the protein is an antibody, the element would be used to test for an antigen analyte (ligand) by utilizing a competitive immunoassay with the ligand and a labeled antigen (ligand analog) competing to bind with the receptor. As disclosed at pages 30-31 of the European Patent, the ligand analog may be added to the test sample or incorporated in a reagent layer in the element that keeps the ligand analog separate from the receptor until the element is contacted with the test sample. The patent discloses at page 46 that the reagent layer may be filter paper. The problem is that such a reagent layer does not provide a uniform concentration of ligand analog and has limited releasability of the ligand analog from the reagent layer during the immunoassay. Furthermore, a physical assembly step is required which can result in additional cost and variability.

The objective of the present invention is to provide an analytical element which can keep components that react with each other separate while at the same time avoiding the problems associated with the use of filter paper for that purpose. That objective is achieved with the present invention which provides an analytical element for the determination of an analyte in an aqueous liquid comprising a first reagent zone comprising a carrier and a first biologically active material and a second reagent zone which is water-soluble and comprises a polymeric material that is naturally soluble in water and a second biologically active material that is interactive with the first biologically active material wherein the first and second reagent zones are contained in a single layer of the element. The element may also include any of a number of other well known zones necessary for determining the analyte in the aqueous sample.

The present invention provides for an analytical element that incorporates two biologically active materials that are interactive with each other, such as a receptor and a ligand analog. The element keeps the two materials separate until the element is contacted with a liquid test sample. The element also provides for good releasability of one of the biologically active materials, ensuring that the competitive reaction in the immunoassay is quickly and adequately established.

FIG. 1 represents an element having first and second reagent zones that are separate layers of the element.

FIG 2 represents an element having first and second reagent zones in separate layers.

FIG. 3 represents an element according to the invention having the first and second reagent zones contained in a single layer of the element. FIG 1 and FIG 2 are presented for comparison with the element of the invention

The element of the invention can be used to determine, either quantitatively or qualitatively, a number of analytes in an aqueous liquid, such as a biological fluid (e.g., whole blood, serum, plasma, urine, spinal fluid, suspensions of human or animal tissue, feces, saliva, lymphatic fluid, and the like). The analytes that the elements of the invention can be used to test for include ligands such as therapeutic drugs (e.g., diphenylhydantoin, phenobarbital, digoxin, theophylline, gentamicin, quinidine, propanolol, tobramycin, lidocaine, procainamide, and the like), natural or synthetic steroids (e.g., cortisol, aldosterone, testosterone, progesterone, estriol, etc.), hormones (e.g., thyroid hormones, peptide hormones, insulin, etc.), proteins (e.g., albumin, IgG, IgM, etc.), antigens, antibodies, and other species that will naturally react with a receptor.

The elements of the invention are useful for determining analytes in aqueous liquid samples and in particular for competitive binding immunoassays, but are not limited to such and can be used in any situation calling for an element having materials that are interactive with each other but are desired to be separated until the analysis is performed.

The first and second biologically active materials of the invention can be any combination of materials that are interactive with each other. These materials are required to react during the assay; however, they must be separated so they do not react before the assay. Materials useful as the first and second biologically active materials include, for example, a ligand analog and receptor (e.g., antigen and antibody), an enzyme and substrate (e.g., glucose oxidase and glucose), or an enzyme inhibitor and an enzyme (e.g., diisopropylfluorophosphate and trypsin). In a preferred embodiment, the first biologically active material is an antibody, which is immobilized on bacteria or on small polymeric particles, and the second biologically active material is an antigen, which may be labeled with, for example, an enzyme.

The first reagent zone of the invention comprises the above-described first biologically active material and a carrier. The carrier can be any of one or more synthetic or natural binder materials (e.g., gelatin or other naturally-occurring colloids, or homopolymers and copolymers, such as poly(acrylamide), poly(vinyl pyrrolidone), poly(N-isopropylacrylamide),poly(acrylamide-co-N-vinyl-2-pyrrolidone), and similar copolymers). In one embodiment, the first reagent zone comprises a porous spreading zone having a suitable porosity for accommodating a test sample (e.g., 1 to 200 $\mu$l), diluted or undiluted. Useful spreading zones can be prepared from paper, porous particulate structures, porous polymeric films, cellulose, wood, glass, fibers, woven and nonwoven fibrous fabrics (synthetic and nonsynthetic) and the like. Materials and processes for making such spreading zones are well known in the art. Useful spreading zones are described in, for example, U.S. Patents 4,292,272; 3,992,158; 4,258,001 and 4,430,436 along with Japanese Patent Publication 57(1982)-101760.

If the first biologically active material of the invention is a receptor, such as an antibody, particularly preferred carrier materials are organo-polymeric particles and a polymeric adhesive, as described in the above-referenced U.S. Patent 4,258,001.

The particles can be composed of a wide variety of organic polymers, including both natural and synthetic polymers, having the requisite properties. Preferably, however, they are composed of one or more addition polymers formed from one or more ethylenically unsaturated polymerizable monomers, such as addition homopolymers of single monomers or copolymers formed from two or more of such monomers.

3

These polymers can be prepared by any of a variety of conventional polymerization methods (e.g. solution, emulsion, dispersion, suspension, etc.). If desired, although the invention is not so limited, the particular polymer can contain one or more reaction sites to link various interactive compositions to the particles.

The polymeric adhesive of U.S. Patent 4,258,001 bonds the organo-polymeric particles to one another to provide a coherent, three-dimensional lattice in the spreading layer. The details of this adhesive are provided in U.S. Patent 4,258,001 noted above. Generally, the adhesive is composed of an organic polymer different from the specific polymer contained in the particles, although quite commonly the adhesive represents a polymer containing many repeating units which are identical or similar to some of those present in the polymer composition of the particles.

Preferably, the adhesive is composed of one or more addition polymers formed from one or more ethylenically unsaturated polymerizable monomers, such as addition copolymers formed from two or more of such monomers. The adhesive can be prepared by any of a variety of conventional polymerization methods.

The second reagent zone of the invention comprises the above-described second biologically active material and a polymeric material that is naturally soluble in water. The second biologically active material is interactive with the first biologically active material (e.g., if the first biologically active material is a receptor, such as an antibody, the second biologically active material may be a ligand analog, such as a labeled antigen) and must be kept separate from and not react with the first biologically active material until the element is contacted with the liquid to be tested.

The polymeric material of the second reagent zone is naturally soluble in water. By "naturally soluble in water," it is means that upon contact with water, the polymer material becomes a homogeneous liquid or solution. Materials that merely absorb water or swell upon contact with water, or materials of which only a portion dissolves upon contact with water, leaving at least some portion of the material in the form of polymeric particles or fibres, are not included within the scope of materials that are "naturally soluble in water." Such materials are more properly classified as water- swellable or water-dispersible. The advantage of using a polymer that is naturally soluble in water as opposed to a polymer that is not water-soluble or only water dispersible is that the biologically active material is rapidly released from the water-soluble layer after contact with the aqueous sample and it immediately available for the competitive binding reaction.

A second advantage is that the water-soluble polymers can easily be coated onto a support or another layer, or into a spreading layer by standard coating methods using standard coating solvents.

Polymeric materials naturally soluble in water that are useful in the present invention are preferably soluble to the extent that a 0.5-25 $\mu$m thick layer of the material will dissolve in less than about 5 minutes when contacted on one side of the layer with water at less than $40^\circ$C. Especially preferred polymeric materials will dissolve in less than 120 seconds when contacted on one side of the layer with water at $37^\circ$C. Examples of polymeric materials that are useful in the present invention include hydroxypropyl cellulose (MW = 60,000-1,000,000), hydroxyethyl cellulose (MW = 100,000-1,000,000), carboxymethyl cellulose, and carboxypropyl cellulose. Other useful polymeric materials include unhardened gelatin, poly-(vinylalcohol), poly(vinylpyrrolidone), poly(acrylamide) or any mixtures or copolymers of the second group.

In addition to the first and second reagent zones, the element of the invention can also comprise any of a number of well known zones or combination of zones needed to determine, either qualitatively or quantitatively, the presence or amount of the analyte being tested. The exact choice and orientation of the various zones will vary according to the particular assay being performed and can easily be determined by one skilled in the art.

For example, in one of the preferred embodiments of the invention, the carrier comprises organo-polymeric beads, which are bound together by an adhesive, the first biologically active material is an antibody immobilized on bacteria or small polymeric beads, the second biologically active material is a labeled antigen interactive with the antibody, and the analyte being tested for is an antigen. In such an element, which is useful in performing an immunoassay, the element might include a third reagent zone comprising an indicator composition that is interactive with the label and other known optional zones such as registration zones for detection of the indicator, radiation-blocking zones and subbing layers.

The exact choice and orientation or configuration of the various reagent zones, spreading zones, registration zones, and others, of the elements of the invention can vary according to the assay being performed and will be known to one skilled in the art. Various elements for assays are described in the above-referenced U.S. Patents 3,992,158 and 4,258,001, and European Patent 66,648. Other elements having different configurations useful for a wide range of assays, which can utilize the present invention, are also well known in the art.

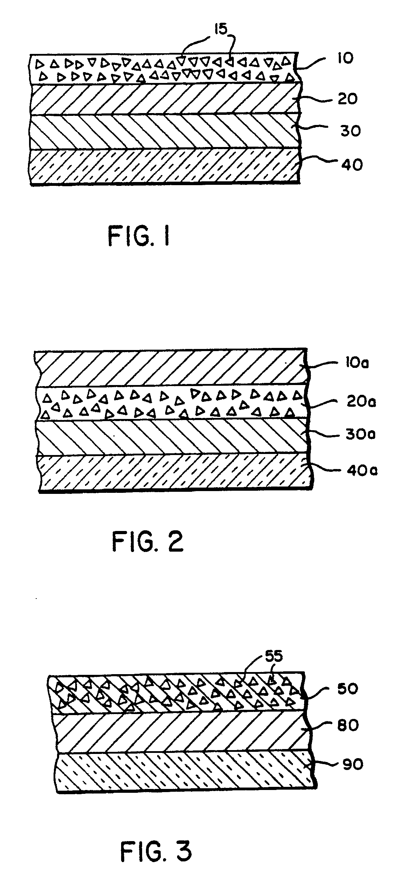

FIG. 1 illustrates an element where the first and second reagent zones each comprise a separate layer of a multilayer analytical element. In FIG. 1, the first reagent zone 10, second reagent zone 20, and a third

4

reagent zone 30 are carried on a support 40. The first reagent zone 10 is preferably a particulate structure spreading zone as described in the above-referenced U.S. Patent 4,258,001, comprising carrier particles 15 5-100 $\mu$m size, preferably 20-40$\mu$m size. A first biologically active material, such as an antibody is also present in the first zone, immobilized on an organism, such as Staphylococcus aureus or small polymeric particles 0.3-10 $\mu$m size, preferably 1 $\mu$m. The second reagent zone 20 comprises a polymeric material that is naturally soluble in water and a second biologically active material, such as a labeled antigen reactive with the immobilized antibody. Third reagent zone 30 comprises a binder such as gelatin and an indicator composition inter- active with the label. Support 40 can be any of a number of well known support materials, such as polyester or cellulose acetate.

FIG. 2 shows an alternative embodiment wherein the first and second reagent zones each comprise a separate layer. The first reagent zone 20a, second reagent zone 10a, and a third reagent zone 30a are carried on a support 40a. The first reagent zone 20a is comprised of a particulate structure, e.g., as described in U.S. Patent 4,258,001, containing a first biologically active material e.g., antibodies immobilized on bacteria or on small polymeric particles. The second reagent zone 10a comprises a polymeric material that is naturally soluble in water and a second biologically active material, e.g., a labeled antigen. The third reagent zone 30a comprises a binder, such as gelatin, and an indicator composition interactive with the label. Support 40a can be any known support material.

FIG. 3 represents an element of the invention where the first and second reagent zones are contained in a single layer of a multilayer analytical element. The first and second reagent zones are contained in the particulate structure spreading layer 50 having organo-polymeric carrier particles 55 (as described in U.S. Patent 4,258,001) and antibody immobilized on S. Aureus or on polymeric particles as described in FIG. 1. The reagent zone 80 comprises a binder such as gelatin and an indicator composition interactive with the label. Support 90 can be any of a number of well known support materials, such as polyester or cellulose acetate. In the above-described drawings, if the label is itself detectable (such as with a fluorescent or radioactive label), and thus does not require an indicator composition with which to react, the third reagent zones 30, 30a or 80 may be replaced with a registration zone.

The element of this invention can utilize any suitable label which can be attached to the ligand to form a ligand analog. Useful labels include radioactive isotopes, fluorescers, enzymes, enzyme inhibitors, allosteric effectors, cofactors and other known enzyme modulators. Enzymes, such as glucose oxidase, peroxidase and alkaline phosphatase, are preferred labels.

When an enzyme label is used, the substrate for the enzyme can be present in the element. Preferably, the substrate can be added to the element prior to or simultaneously with the liquid sample, or after completion of the binding reaction. It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. If the enzyme label is glucose oxidase, the substrate is glucose. Glucose can be added to the element or present in the element in an amount of at least 30 mMolar, and preferably from 50 to 200 mMolar. A worker skilled in the art would know how to adjust the amount of a particular substrate for the amount of enzyme label used in the assay.

When certain labels are used, e.g. enzymes, cofactors or enzyme modulators, a third reagent zone preferably contains an indicator composition comprising one or more reagents which provide a detectable species as a result of reaction of the label. Preferably, the indicator composition is a colorimetric indicator composition which provides a colorimetrically detectable species as a result of enzymatic reaction of an enzyme-labeled ligand analog with a substrate. The indicator composition can be a single compound which produces a detectable dye upon enzymatic reaction, or a combination of reagents which produce the dye. For example, when glucose is used as the substrate and glucose oxidase as the enzyme label, the colorimetric indicator composition can include a coupler, such as 7-hydroxy-1-naphthol, and an oxidizable compound, such as 4-aminoantipyrine. Preferably, the composition can include a leuco dye and peroxidase or another suitable peroxidative compound. Useful leuco dyes are known in the art and include those, for example, described in U.S. Patent 4,089,747 and European Patent Application 0 162 685. The particular amounts of the colorimetric indicator composition and its various components are within the skill of a worker in the art.

The zones of the element can contain a variety of other desirable but optional components, including surfactants, thickeners, buffers, hardeners, antioxidants, coupler solvents, and other materials known in the art. The amounts of these components are also within the skill of a worker in the art.

When the first and second reagent zones are separate layers in a multilayer analytical element, the element is prepared according to techniques well known in the art, such as described in the above-referenced U.S. Patents 3,992,158 and 4,258,001. The layer of naturally soluble polymeric material can be coated as a layer in the element by known means. When the first and second reagent zones are contained in a single layer of an analytical element in accordance with the invention, a preferred method of preparing that layer is to first prepare a particulate spreading layer (as described in U.S. Patent 4,258,001), having a first biologically active material such as an antibody immobilized on the surfaces of bacteria or particles. Over that layer is coated a solution of a water-soluble polymeric material and a second biologically active material such as a labeled antigen that is reactive with the antibody. This coating step is preferably performed so that the water-soluble polymer spreads into the spreading layer during the coating operation, coating the polymer particles in a way such that the two biologically active materials do not react. This is accomplished by a standard coating operation using the water-soluble polymers of this invention.

The elements of the invention are useful in performing any of a number of well known analyses in which it is desirable to have present a plurality of biologically active materials that are interactive with each other, but should only react when the element has been contacted with an aqueous test sample. The element is particularly useful in performing heterogeneous immunoassays, as described in European Patent Application 0 209 378, or homogeneous immunoassays.

The method of using the element of the invention will depend upon the type of analysis being performed. Such methods are well known in the art and generally involve physically contacting the element, preferably a spreading zone of the element, with an aqueous liquid sample (e.g., 1 to 200 $\mu$l) so that the liquid sample mixes with the biologically active components and other reagents in the element. The contacting is done in the presence of an indicator composition for providing a detectable change in response to the presence of the analyte being tested for. After contacting, the presence or amount of the analyte is determined as a function of the detectable change. Reagents can also be added simultaneously or sequentially with the liquid sample.

In one use of an element according to the invention as described in the FIGURES, contact of a sample is done in such a manner that reaction of antibodies (receptor) with a drug (ligand) or conjugate (ligand analog) occurs during sample introduction along with substantial horizontal separation of uncomplexed and complexed analyte. This contact can be carried out by hand or with a machine using a pipette or other suitable dispensing means to dispense the test sample. The sample of liquid can be applied to the element spreading layer in a number of ways to effect horizontal separation. For example, a relatively large liquid sample (e.g. up to 200 $\mu$l) can be applied slowly (e.g. over at least 5 seconds) in a continuous manner using a pipette, capillary tube or other means. Alternatively, the sample can be applied in small portions, e.g. as a series of two or more droplets (e.g. 0.1 to 1 $\mu$l) over a period of time (e.g. over at least 5 seconds).

In a preferred embodiment, horizontal separation can be accomplished by slowly adding a wash fluid after the liquid sample has been applied to the element. This wash causes unbound materials (i.e. drug and labeled conjugate) to move away from the bound materials. The wash fluid can contain a buffer and any other reagents that are described, e.g. an enzyme substrate.

After sample application in either embodiment, the element is exposed to any conditioning, such as incubation or heating, that may be desirable to quicken or otherwise facilitate obtaining the test result.

The following examples are provided to illustrate the practice of the present invention.

Example 1

An analytical element for the determination of phenobarbital was prepared having the format and components shown below. Except as otherwise noted, suitable ranges of quantities together with the specific quantity used are parenthetically given in grams per square meter.

## Spreading Layer:

— Poly(m+p-vinyltoluene-co-p-t-butylstyrene-co-methacrylic acid) beads (20-40 μm) (50-200, 130)

— Poly(methylacrylate-co-2-acrylamido-2-methyl propane sulfonic acid-co-2-acetoacetoxyethyl methacrylate) (1-10, 4)

— Triton X-100® surfactant (0.1-12, 11)

— Water (0.005-0.1, 0.01)

— S. Aureus coated with phenobarbital antiserum (1-10, 6)

## Water-Soluble Layer:

— Poly(acrylamide-co-N-vinyl-2-pyrrolidone) (0.1-10, 1)

— Zonyl FSN surfactant (0.01-2, 0.1)

— 5-Ethyl-5-phenylhydantoin valerate-glucose oxidase (0.0005-0.1, 0.001)

## Reagent Layer:

— Gelatin (hardened) (1-20, 11)

— 4'-Hydroxyacetanilide (0.005-0.1, 0.05)

— Sodium dodecyl sulfate (0.5-10, 2)

— 5,5-Dimethyl-1,3-cyclohexanedione (0.01-0.5, 0.05)

— 4,5-bis(4-Dimethylaminophenyl)-2-(4-hydroxy-3,5-dimethoxyphenyl)imidazole (0.02-1, 0.16)

— Peroxidase (100-5000 $IU/m^2$, 1000 $IU/m^2$)

/ / / / Poly(ethylene terephthalate) Support / / / / / /

The 5-ethyl,5-phenyl-hydantoin-valeric acid hapten used to prepare the conjugate of Example 1 below was obtained from a reaction of 5-bromo-methyl valerate and 5-ethyl,5-phenyl-hydantoin after hydrolysis of the resulting ester. The 5-ethyl,5-phenyl-hydantoin derivative was prepared by a known procedure. The 5-ethyl,5-phenyl-hydantoin-valeric acid compound was then coupled to an amine attached to a label by the mixed anhydride procedure, generating amide bonds.

A series of test samples containing various amounts of phenobarbital were prepared in solutions comprising 0.01 M 3-(N-morpholino)propanesulfonic acid (MOPS) buffer (pH 7.0), 0.15 M sodium chloride and 0.10% bovine serum albumin (BSA). The concentrations of phenobarbital in the test samples are listed in Table I.

A ten microliter sample of each test sample was spotted onto a finite area of the above element and the element was incubated for 5 minutes at 37°C.

A ten microliter amount of wash fluid comprising 0.01 M MOPS (pH 7.0), 0.15 M sodium chloride, 0.10 M glucose and 0.10% BSA was spotted over each of the original spots and the element was incubated for 2-5 minutes at 37° C. The reflection density at 670 nm was measured in the center of the finite area using a standard reflectometer. Transmission densities ($D_T$) were then calculated using the Williams-Clapper transformer (J. Optical Soc. Am. 43, 595: 1953).

Results are shown in Table I as the change in $D_T$ with time, which values are inversely proportional to the phenobarbital concentration.

TABLE I

| Determination of Phenobarbital | |
|---|---|
| Phenobarbital Conc. (g/mL) | Rate ($D_T$/minute) |
| 0 | 0.062 |
| 1 | 0.055 |
| 2 | 0.053 |
| 4 | 0.047 |
| 8 | 0.043 |
| 16 | 0.036 |
| 32 | 0.032 |
| 64 | 0.026 |
| 128 | 0.022 |

Example 2

An analytical element for the determination of phenytoin was prepared as described in Example 1, except that both the Water-soluble Layer and the Reagent Layer additionally contained MOPS buffer (0.1-8 g/m², 1.1 g/m²)

A series of test samples containing various amounts of phenytoin in a human serum-based fluid were prepared. The concentrations of phenytoin in the test samples are listed in Table II.

A ten microliter sample of each test sample was spotted onto a finite area of the above element and the element was incubated for 5 minutes at 37° C.

A ten microliter amount of a wash fluid comprising 0.01 M MOPS buffer (pH 7.0), 0.15 M sodium chloride and 0.10 M glucose was spotted over each of the original spots and the element was incubated at 37° C for 2 minutes. The reflection density was measured in the center of the finite area at 670 nm using a conventional reflectometer. Transmission densities were then calculated using the Williams-Clapper transformer.

Results are shown in Table II as the change in DT with time, which values are inversely proportional to the phenytoin concentration.

TABLE II

| Determination of Phenytoin | |
|---|---|
| Phenytoin Conc. (g/mL) | Rate ($D_T$/minute) |
| 0 | 0.180 |
| 1 | 0.172 |
| 2 | 0.164 |
| 4 | 0.153 |
| 8 | 0.141 |
| 16 | 0.129 |
| 64 | 0.108 |
| 128 | 0.093 |

Example 3

An analytical element for the determination of phenobarbital was prepared by coating a water-soluble layer comprising hydroxyethyl cellulose ($0.05-5$ g/m², $0.5$ g/m²) and phenobarbital-glucose oxidase conjugate ($0.0005-0.1$ g/m², $0.001$ g/m²) over an element having the following format and composition. Except as otherwise noted quantities are parenthetically given in grams per square meter.

---

## Spreading Layer:

— Polystyrene beads coated with normal rabbit serum (25–200, 140)

— Zonyl FSN® surfactant (0.1–2.5, 1.0)

— Poly(n–butylacrylate–co–styrene–co–2–acrylamido– 2–methylpropane sulfonic acid sodium salt) (1–20, 8)

— S. Aureus coated with phenobarbital antiserum (0.1–5, 4)

---

## Reagent Layer:

— Gelatin (hardened) (1–20, 3)

— Leuco dye (0.025–0.6, 0.3)

— 2,4–Di–n–amylphenol (0.9–4, 2)

— Dimedone® (0.05–5, 1.02)

— Alkanol XC® (0.01–0.2, 0.07)

— $KH_2PO_4$, pH 7.0 (1–2, 1.5)

— Peroxidase (500–10,000 $IU/m^2$, 5000 $IU/m^2$)

---

/ /                                                   / /
/   /  Poly(ethylene terephthalate) Support  /   /
/   /                                               /   /

---

Four identical control solutions, each containing 100 mM glucose were spotted onto samples of the above element. The elements were incubated at 37° C for 2-3 minutes while the reflection densities at 670 nm were measured in a modified conventional reflectometer. The average rate of dye formation ($\Delta$ DR/minute) was then calculated.

Four identical test solutions, each containing 100 mM glucose and $10^{-3}$ M phenobarbital solution were also spotted onto samples of the same element and the reflection densities were measured as above. The average rate of dye formation was also calculated.

A difference of rate was found to exist between the test solutions containing phenobarbital and substrate and the control solutions containing only substrate, indicating that this element can be used for the determination of phenobarbital.

## Claims

1. An analytical element for the determination of an analyte in an aqueous liquid comprising:
   a first reagent zone comprising a carrier and a first biologically active material; and
   a second reagent zone comprising a second biologically active material that is interactive with the first biologically active material,

characterised in that the first and second reagent zones are contained in a single layer in said element and in that the second reagent zone is water soluble and comprises a polymeric material that is naturally soluble in water.

2. The element of claim 1 wherein the carrier comprises polymeric particles, which are held in a three-dimensional lattice by a polymer adhesive different from that of said particles, the first biologically active material is immobilized on bacteria or small polymeric particles, and the second biologically active material is dispersed in said polymeric material that is naturally soluble in water, which polymeric material is dispersed as a film on said polymer particles or bacteria.

3. The element of claim 2 wherein the first biologically active material is an antibody and the second biologically active material is a labeled antigen interactive with the antibody.

4. The element of claim 1 wherein at least one of the first and second biologically active materials is interactive with the analyte in the aqueous sample.

5. The element of claim 4 wherein the first biologically active material is interactive with the analyte in the aqueous sample.

6. The element of claim 5 wherein the carrier comprises polymeric particles, the first biologically active material is an antibody immobilized on bacteria or small polymeric particles, and the second biologically active material is a labeled antigen interactive with the antibody.

7. The element of any one of the preceding claims wherein the polymeric material that is naturally soluble in water is soluble to the extent that a 0.5-25 $\mu$m thick layer of the polymeric material will dissolve in less than 5 minutes when contacted on one side of the layer with water at a temperature less than 40°C.

8. The element of claim 7 wherein the polymeric material is hydroxyethyl cellulose, hydroxypropyl cellulose, carboxymethyl cellulose, carboxypropyl cellulose, unhardened gelatin, poly(vinylalcohol), poly(vinylpyrrolidone) or poly(acrylamide).

**Revendications**

1. Produit analytique pour la détermination d'une substance dans un liquide aqueux comprenant :
une première zone à réactif contenant un milieu porteur et une première substance ayant une activité biologique, et
une seconde zone à réactif contenant une seconde substance ayant une activité biologique, interactive avec la première substance à activité biologique,
caractérisé en ce que la première et la seconde zones à réactif sont contenues dans une couche unique du produit et en ce que la seconde zone à réactif est soluble dans l'eau et comprend un polymère naturellement soluble dans l'eau.

2. Produit de la revendication 1, dans lequel le milieu porteur comprend des particules de polymère qui sont maintenues dans un réseau tridimensionnel par un adhésif polymère différent du polymère des particules, la première substance à activité biologique est immobilisée sur des bactéries ou sur des petites particules de polymère, et la seconde substance à activité biologique est dispersée dans ledit polymère naturellement soluble dans l'eau, lequel polymère étant dispersé sous forme d'un film sur les particules de polymère ou sur les bactéries.

3. Produit de la revendication 2, dans lequel la première substance ayant une activité biologique est un anticorps et la seconde substance ayant une activité biologique est un antigène marqué interactif avec l'anticorps.

4. Produit de la revendication 1, dans lequel au moins l'une des première et seconde substances à activité biologique est interactive avec la substance à analyser dans le liquide aqueux.

5. Produit de la revendication 4, dans lequel la première substance à activité biologique est interactive

10

EP 0 253 581 B1

avec la substance à analyser dans le liquide aqueux.

6. Produit de la revendication 5, dans lequel le milieu porteur comprend des particules de polymère, la première substance à activité biologique est un anticorps immobilisé sur des bactéries ou sur des petites particules de polymère et la seconde substance à activité biologique est un antigène marqué interactif avec l'anticorps.

7. Produit de l'une des revendications précédentes, dans lequel le polymère naturellement soluble dans l'eau a une solubilité telle qu'une couche de 0,5-25 $\mu$m d'épaisseur de ce polymère se dissout en moins de 5 minutes lorsqu'une des faces de cette couche est en contact avec de l'eau au moins à une température de 40° C.

8. Produit de la revendication 7, dans lequel le polymère est l'hydroxyethyl cellulose, l'hydroxypropyl cellulose, la carboxymethyl cellulose, la carboxypropyl cellulose, la gélatine non tannée, le poly-(alcoolvinylique), la poly(vinylpyrrolidone) ou le poly(acrylamide).

**Patentansprüche**

1. Analytisches Element für die Bestimmung eines Analyten in einer wäßrigen Flüssigkeit mit:

einer ersten Reagenszone mit einem Träger und einem ersten biologisch aktiven Material und

einer zweiten Reagenszone mit einem zweiten biologisch aktiven Material, das mit dem ersten biologisch aktiven Material zu reagieren vermag,

dadurch gekennzeichnet, daß sich die erste und die zweite Reagenszone in einer Schicht in dem Element befinden und daß die zweite Reagenszone wasserlöslich ist und ein polymeres Material enthält, das von Natur aus in Wasser löslich ist.

2. Element nach Anspruch 1, in dem der Träger Polymerteilchen umfaßt, die von einem Polymerklebstoff, der von dem Polymer der Teilchen verschieden ist, in einem dreidimensionalen Gitter zusammengehalten werden, in dem das erste biologisch aktive Material auf Bakterien oder kleinen Polymerteilchen immobilisiert ist und in dem das zweite biologisch aktive Material in dem polymeren Material dispergiert vorliegt, das von Natur aus in Wasser löslich ist, wobei das polymere Material als Film auf den Polymerteilchen oder Bakterien dispergiert ist.

3. Element nach Anspruch 2, in dem das erste biologisch aktive Material ein Antikörper und das zweite biologisch aktive Material ein markiertes Antigen ist, das mit dem Antikörper zu reagieren vermag.

4. Element nach Anspruch 1, in dem mindestens eine der ersten und zweiten biologisch aktiven Materialien mit dem Analyten in der wäßrigen Probe zu reagieren vermag.

5. Element nach Anspruch 4, in dem das erste biologisch aktive Material mit dem Analyten in der wäßrigen Probe zu reagieren vermag.

6. Element nach Anspruch 5, in dem der Träger Polymerteilchen aufweist, in dem das erste biologisch aktive Material ein Antikörper ist, der auf Bakterien oder kleinen Polymerteilchen immobilisiert ist, und in dem das zweite biologisch aktive Material ein markiertes Antigen ist, das mit dem Antikörper zu reagieren vermag.

7. Element nach einem der vorstehenden Ansprüche, in dem das polymere Material, das von Natur aus in Wasser unlöslich ist, in dem Masse löslich ist, daß sich eine 0,5 - 25 $\mu$m dicke Schicht des polymeren Materials in weniger als 5 Minuten löst, wenn eine Seite der Schicht mit Wasser bei einer Temperatur von weniger als 40° C in Kontakt gebracht wird.

8. Element nach Anspruch 7, in dem das polymere Material Hydroxyethylcellulose, Hydroxypropylcellulose, Carboxymethylcellulose, Carboxypropylcellulose, ungehärtete Gelatine, Poly(vinylalkohol), Poly-(vinylpyrrolidon) oder Poly(acrylamid) ist.

11

15

10

20

30

40

## FIG. 1

10a

20a

30a

40a

## FIG. 2

55

50

80

90

## FIG. 3